# EUROPEAN PATENT APPLICATION

(11) **EP 1 293 512 A2**
(43) Date of publication of application: **19.03.2003**
(21) Application number: 02256292.0
(22) Date of filing: 11.09.2002
(51) Int. Cl.: C07K 14/72, C12N 15/12, C12N 5/10, G01N 33/50, A61K 38/17

(54) **DNA encoding the baboon and cynomolgus macaque prostaglandin E2 receptor EP4 subtype**

(30) Priority: 17.09.2001 US 322916 P
(71) Applicant: Pfizer Products Inc., Groton, CT 06340-5146 (US)
(72) Inventor: Castleberry, Tessa Ann, Groton, Connecticut 06340 (US); Lu, Bihong, Groton, Connecticut 06340 (US); Owen, Thomas Allen, Groton, Connecticut 06340 (US); Smock, Steven Lee, Groton, Connecticut 06340 (US)
(74) Representative: Hayles, James Richard

(57) **Abstract**

The present invention provides isolated baboon and cynomolgus macaque EP4 protein, DNA molecules encoding the protein, vectors and cells carrying the DNA molecules, specific bind partners for the protein, and methods of determining ligands for baboon and cynomolgus macaque EP4.

## Description

### FIELD OF THE INVENTION

The present invention relates to baboon (*Papio cynocephalus)* prostaglandin E₂ receptor EP4 subtype (baboon EP4 or baEP4 herein) corresponding to that found in baboon tissues, as well as to cynomolgus macaque (*Macaca fascicularis)* prostaglandin E₂ receptor EP4 subtype (cynomolgus macaque EP4 or cynEP4 herein) corresponding to that found in cynomolgus macaque tissues. Collectively, these two receptors are referred to as baboon/cyno EP4 or ba/cynEP4 herein. In other aspects, the invention relates to, inter alia, structural variants of ba/cynEP4, polynucleotides that encode ba/cynEP4 and its structural variants, expression vectors containing the polynucleotides, and cells transformed by the expression vectors.

### BACKGROUND OF THE INVENTION

Prostaglandin E₂ (PGE₂) is an important mediator of diverse biological functions in many tissues. PGE₂ binds with high affinity to four cell surface receptors (EP1-EP4) which are members of the superfamily of G protein-coupled receptors. Cloning of the cDNAs encoding the EP4 receptor from several species has revealed a highly conserved molecule with 7 potential transmembrane domains and a long intracellular carboxy-terminal region. As with the reported EP2 receptors, the EP4 receptors are functionally coupled to adenylate cyclase, resulting in elevated intracellular cyclic adenosine 5' monophosphate (cAMP) levels upon activation. The human EP4 receptor complementary DNA encodes a 488 amino acid polypeptide with a predicted molecular mass of approximately 53 kDa (Bastien et al., *J. Biol. Chem.,* 269:11873-77 (1994)). Care must be taken in reviewing the literature before 1995, when this receptor was generally referred to as the EP2 receptor (Nishigaki et al., *FEBS Lett.,* 364:339-41 (1995)). In addition to the human receptor, EP4 receptors for the mouse, rat, rabbit, and dog have been cloned (Boie et al., *Eur. J. Pharmacol.,* 340:227-41 (1997); Breyer et al., *Am. J. Physiol.*, 270:F485-93 (1996); Nishigaki et al., *FEBS Lett.,* 364:339-41 (1995); Bastien et al., *J. Biol. Chem.,* 269:11873-77 (1994); An et al., *Biochem. Biophys. Res. Comm.,* 197:263-70 (1993); Honda et al., *J. Biol. Chem.,* 268:7759-62 (1993); Castleberry et al., *Prostaglandins,* 65:167-187 (2001)).

EP4 receptors may be pharmacologically distinguished from the EP1 and EP3 receptors by their insensitivity to sulprostone and from EP2 receptors by their insensitivity to butaprost (Kiriyama et al., *Br. J. Pharmacol.,* 122:217-24 (1997); Boie et al., *Eur. J. Pharmacol.,* 340:227-41 (1997)) and relatively selective activation by prostaglandin E₁-OH (Id).

The prostaglandins are a group of widely distributed fatty acid metabolites that exert a tremendous variety of physiological effects in most mammalian tissues. These molecules are either chemically unstable or very rapidly metabolized and are, therefore, believed to primarily affect local physiological events close to their site of synthesis. Prostaglandin E₂ (PGE₂) is formed from arachidonic acid through the actions of cyclooxygenase and PGE synthase. The biological actions of PGE₂ on diverse tissues, including bone, are mediated through at least four pharmacologically distinct G protein-coupled cell surface receptors designated EP1, EP2, EP3, and EP4 on the basis of their responses to agonists and antagonists. Activation of the EP1 receptor results in increased intracellular calcium levels via phospholipase C, while EP3 activation results in reduced intracellular cAMP due to inhibition of adenylate cyclase. EP2 and EP4 receptors stimulate adenylate cyclase leading to elevated levels of intracellular cAMP. The cloning of various human, mouse, rat, dog, and rabbit EP receptor subtypes over the past several years has led to a greater understanding of the mechanisms of PGE₂ action.

Prostaglandins play a role in bone metabolism by regulating both formation and resorption of bone. The stimulation of bone formation in organ cultures appears to be due to an increase in both the replication and differentiation of osteoblast precursors (Gronowicz et al., *Exp. Cell Res.,* 212:314-20 (1994), Raisz and Fall, *Endocrinology,* 126:1654-59 (1990)). The anabolic actions of PGE₂ in bone are believed to occur as a result of local autocrine or paracrine production of PGE₂ in response to mechanical forces, cytokines, growth factors, systemic hormones, and to prostaglandins themselves (Raisz, *J. Nutr.,* 125:2024S-2027S (1995)) and have been linked to increased levels of cAMP, implicating either the EP2 or EP4 receptor subtype.

### SUMMARY OF THE INVENTION

To further the understanding of the molecular and biochemical actions of prostaglandins in bone, as well as to expand the understanding of the cAMP linked prostaglandin receptors, we have cloned, expressed, and characterized the baboon and cynomolgus macaque EP4 receptors. These EP4 receptors have functional characteristics in common with the previously reported EP4 receptors from human, mouse, rabbit, dog, and rat with respect to ligand binding and activation.

Until the work by the present inventors, no work had been reported on isolating a factor having baEP4 activity from baboons, or cynEP4 activity from cynomolgus macaques. Several potential difficulties had to be surmounted before the present inventors succeeded in achieving the present invention. First, although EP4 proteins had been isolated from five other species, as mentioned above, it was unclear to what extent there would be homology between these known EP4 materials and that from baboons or cynomolgus macaques. Accordingly, it was unclear whether primers based on EP4 proteins associated with other species would be useful in isolating material from baboons or cynomolgus macaques. Second it was unclear whether baboon or cynomolgus macaque tissues would produce sufficient amounts of messenger RNA (mRNA) for cloning the receptor.

In spite of the above uncertainties and other difficulties, the present inventors succeeded in isolating full cDNA sequences corresponding to proteins having ba/cynEP4 activity from both baboons and cynomolgus macaques. This discovery established the basis of the present invention.

The protein-coding regions of baboon and cynomolgus macaque EP4 were contained in open reading frames of 1470 bp and encode identical proteins of 490 amino acids with a predicted molecular weight of 53.4 kD. Sequence analysis of this open reading frame reveals >97% identity to the human EP4 cDNA protein coding region when both the nucleotide and protein sequences are compared. Following transient transfection of baEP4 and cynEP4 into CHO-K1 cells, competition binding studies using ³H-PGE₂ as ligand demonstrated specific displacement by PGE₂ and 7-[2-(3-hydroxy-4-phenyl-butyl)-5-oxo-pyrrolidin-1 -yl]-heptanoic acid (an EP4-specific ligand, hereinafter referred to as EP4SL) with IC50 values of 2.6 nM for baEP4, 2.5 nM for cynEP4 and 361 nM for baEP4, 300 nM for cynEP4, respectively. Treatment with PGE₂ or EP4SL also resulted in increased levels of cAMP in EP4 transfected cells with EC50 values of 22.4 nM for baEP4, 8.9 nM for cynEP4 and 872 nM for baEP4, 560 nM for cynEP4, respectively, but not in parental CHO-K1 cells. In contrast, butaprost, an EP2 selective ligand, and sulprostone, an EP1/EP3 selective ligand, did not bind to these receptors.

Thus, baboon and cynomolgus macaque cDNAs have been isolated that encode an open reading frame with a DNA and predicted protein sequence that is highly homologous to the reported human PGE₂ receptor EP4 subtype. When the baboon or cynomolgus macaque EP4 cDNA is expressed in CHO-K1 cells, the encoded protein exhibits ligand binding properties characteristic of the EP4 subtype and when activated by appropriate ligands, these receptors cause the accumulation of intracellular cAMP.

The present invention has several aspects. In a first aspect, the present invention relates to isolated proteinaceous molecules having an activity of ba/cynEP4 and comprising an amino acid sequence corresponding to SEQ ID NO:3.

In another aspect, the present invention relates to isolated DNA molecules encoding the proteinaceous molecules described above (see, for example, SEQ ID NO:1).

Other aspects of the present invention relate to recombinant expression vectors capable of transferring the recited polynucleotide molecules to suitable host cells, and to cells transformed by these expression vectors.

In another aspect the present invention relates to methods of producing the recited proteinaceous molecules by recombinant means.

In a further aspect of the present invention, it relates to pharmaceutical compositions comprising the recited proteinaceous molecules.

In yet another aspect, the present invention relates to specific binding partners to the recited proteinaceous molecules.

In still another aspect, the present invention relates to methods of determining ligands for ba/cynEP4.

The above aspects of the present invention enable production of large quantities of proteinaceous molecules having ba/cynEP4 activity. It is contemplated that such molecules can be used, e.g., for veterinary purposes to treat baboons and cynomolgus macaques, as described in greater detail hereinbelow.

### DESCRIPTION OF THE DRAWINGS

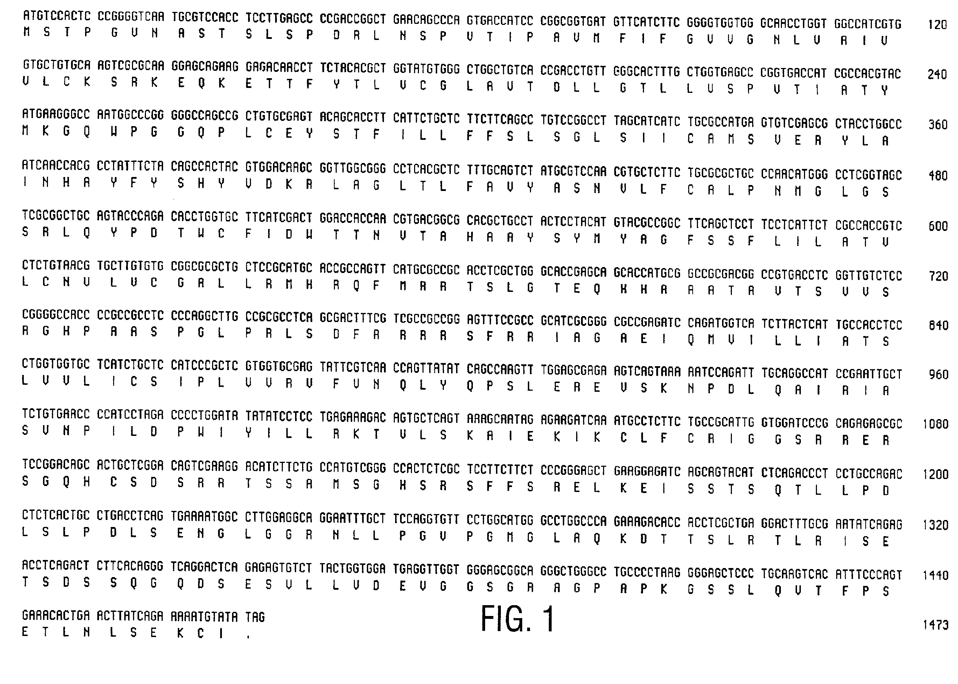

FIGURE 1 provides the nucleotide and predicted protein sequence of baboon EP4. This clone has an open reading frame of 1470 bp. The predicted amino acid sequence of the open reading frame is shown in single letter format below the DNA sequence.

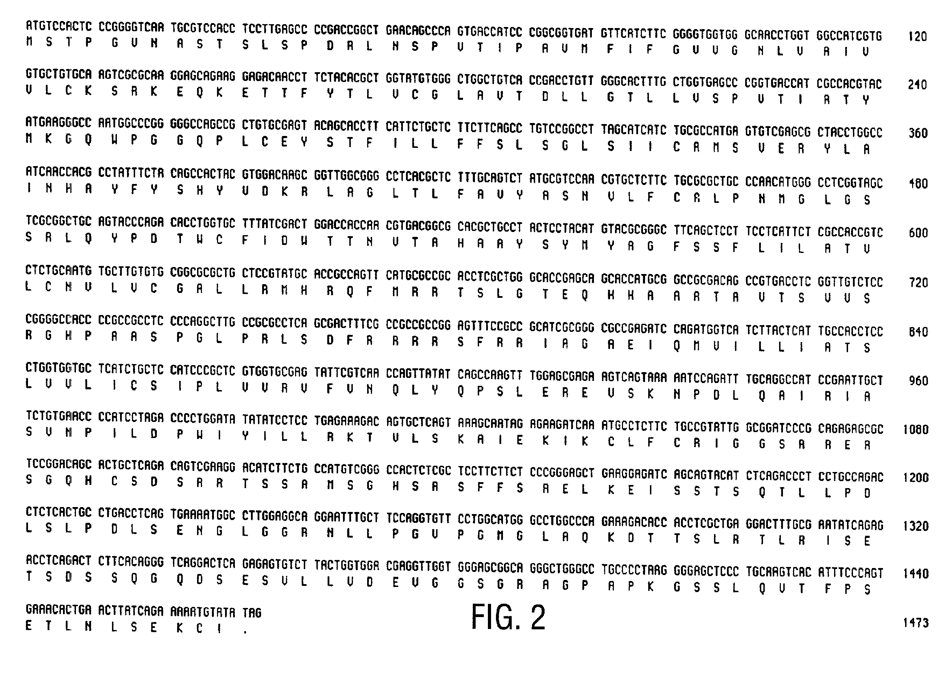

FIGURE 2 provides the nucleotide and predicted protein sequence of cynomolgus macaque EP4. This clone has an open reading frame of 1470 bp. The predicted amino acid sequence of the open reading frame is shown in single letter format below the DNA sequence.

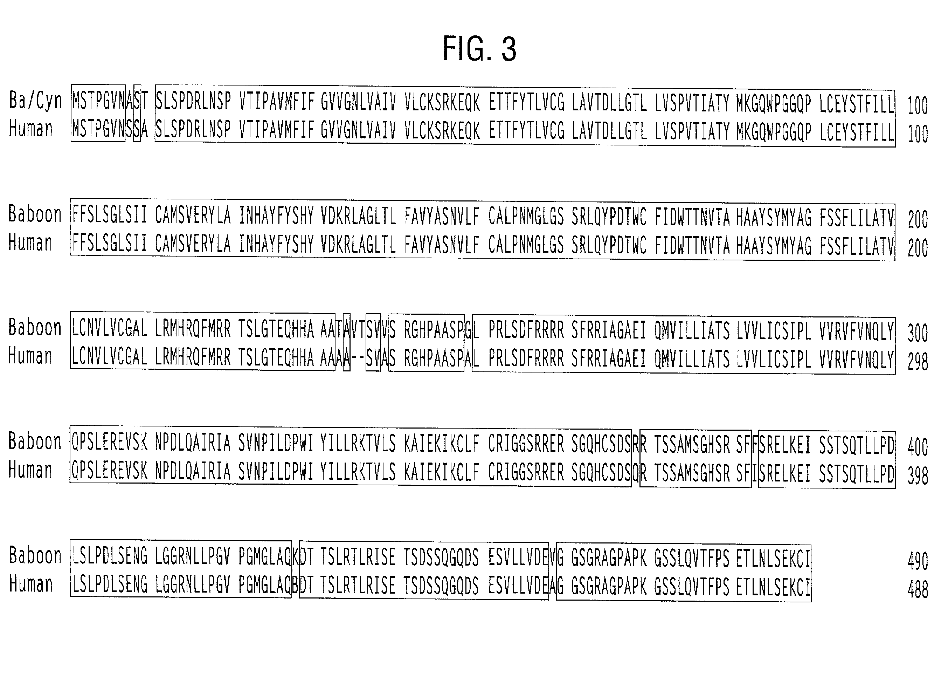

FIGURE 3 provides a comparison of the predicted protein sequences of the ba/cynEP4 open reading frame with that reported for human EP4. Alignment of these protein sequences revealed >97% identity. Nucleotides identical between the two species are boxed and gaps have been introduced where necessary for alignment.

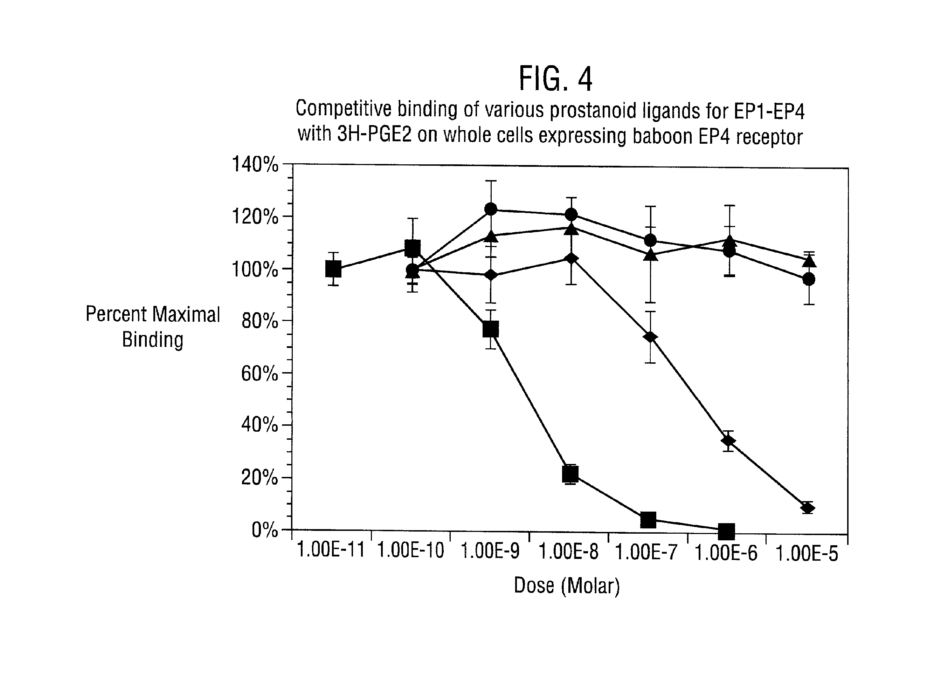

FIGURE 4 provides a characterization of ligand binding to CHO cells expressing baboon prostaglandin receptor EP4. Competitive binding of various prostanoid ligands for EP1-EP4 with ³H-PGE₂ on CHO cells transiently transfected with baboon EP4. Each indicated concentration of (■) PGE₂, (◆) EP4SL, (▲) butaprost, or (•) sulprostone was incubated with three wells of a 24-well dish of CHO cells transiently transfected with baboon EP4 for 1 hr on ice in the presence of 3 nM ³H-PGE₂. Following washing and solubilization of the cells, specific binding was determined by scintillation counting. Error bars represent one standard deviation. Butaprost was obtained from Dr. Harold Kluender (Bayer, Inc., New Haven, CT). EP4SL was produced at Pfizer, Inc. in Groton, CT. All other prostanoid compounds were purchased from Cayman Chemical (Ann Arbor, MI).

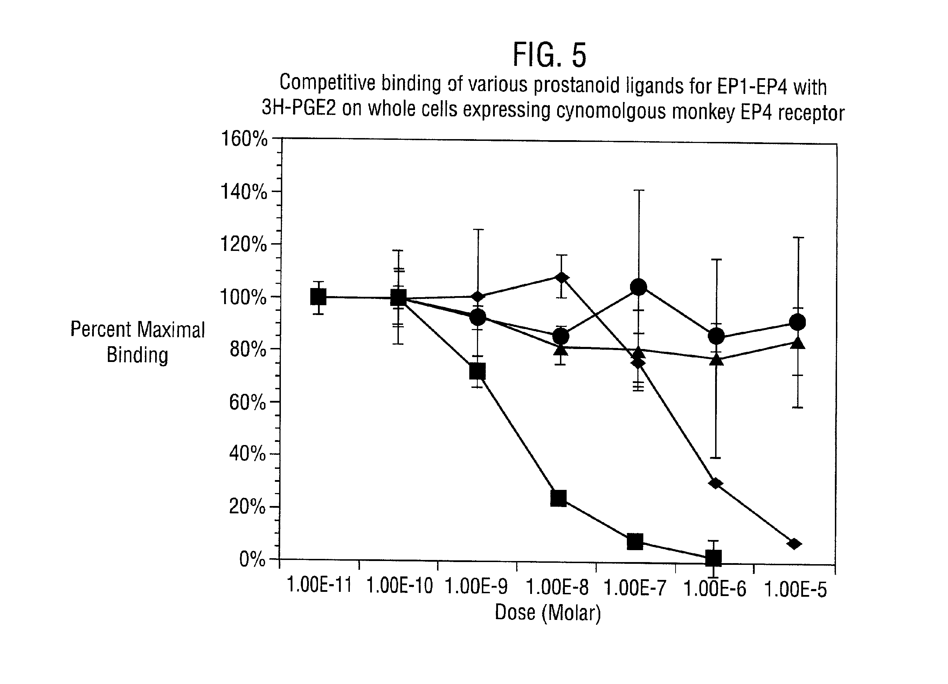

FIGURE 5 provides a characterization of ligand binding to CHO cells expressing cynomolgus macaque prostaglandin receptor EP4. Competitive binding of various prostanoid ligands for EP1-EP4 with ³H-PGE₂ on CHO cells transiently transfected with cynEP4. Each indicated concentration of (■) PGE₂, (◆) EP4SL, (▲) butaprost, or (•) sulprostone was incubated with three wells of a 24-well dish of CHO cells transiently transfected with cynEP4 for 1 hr on ice in the presence of 3 nM ³H-PGE₂. Following washing and solubilization of the cells, specific binding was determined by scintillation counting. Error bars represent one standard deviation.

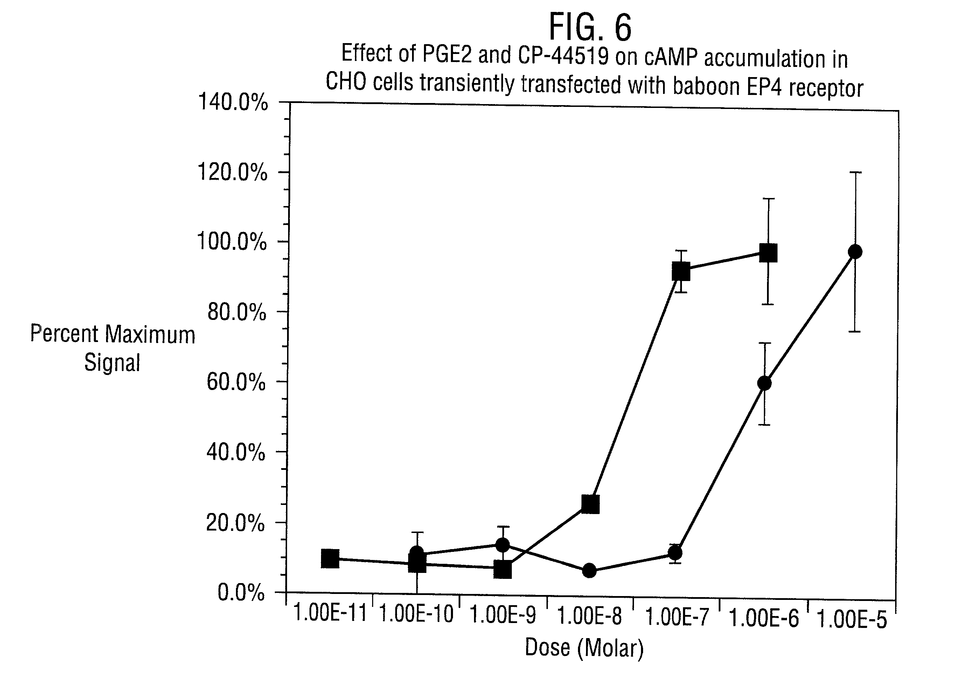

FIGURE 6 provides characterization of secondary signaling in CHO cells expressing baboon prostaglandin receptor EP4. An SPA assay was performed on CHO cells transiently transfected with baboon EP4 following treatment for 12 minutes with either (■) PGE₂ or the EP4 specific ligand (•) EP4SL. Error bars represent one standard deviation.

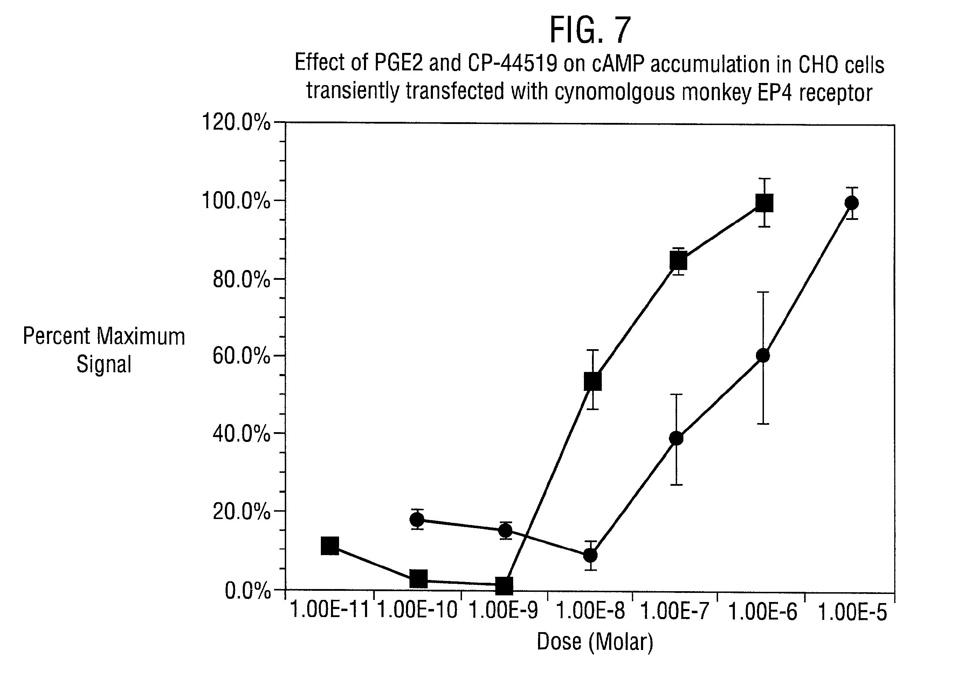

FIGURE 7 provides characterization of secondary signaling in CHO cells expressing cynomolgus macaque prostaglandin receptor EP4. An SPA assay was performed on CHO cells transiently transfected with cynEP4 following treatment for 12 minutes with either (■) PGE₂ or the EP4 specific ligand (•) EP4SL. Error bars represent one standard deviation.

### DETAILED DESCRIPTION OF THE INVENTION

The screening of baboon spleen and heart cDNA libraries by PCR using degenerate primers designed based on the DNA sequences encoding the human, rat, mouse, dog, and rabbit EP4 receptor protein-coding regions resulted in the cloning of an open reading frame of 1470 bp (see SEQ ID NO:1). A reverse transcriptase-polymerase chain reaction (RT-PCR) strategy was used to clone the cynEP4 from cynomolgus macaque spleen and heart RNA. This strategy employed both random hexamers and oligo-dT primers for the reverse transcription and, for the PCR phase, degenerate primers designed based on the DNA sequences encoding the human, rat, mouse, dog, and rabbit EP4 receptor protein-coding regions. This resulted in the cloning of an open reading frame of 1470 bp for the cynEP4 gene (see SEQ ID NO:2). These cloned DNAs are capable of encoding proteins of 490 amino acids with a predicted molecular weight of 53,463 daltons (see SEQ ID NO:3). Analysis of the predicted protein by the method of Kyte and Doolittle (Kyte and Doolittle, *J. Mol. Biol.* 157:105-32 (1983)) indicated the presence of seven hydrophobic sequences, consistent with the presence of seven transmembrane domains typical of this family of G protein-coupled receptors.

Comparison of the cDNA and predicted protein sequences of the ba/cynEP4 open reading frame with those reported for human EP4 revealed >97% identity at the DNA level (data not shown) and >97% identity at the protein level (Fig. 3). While the entire EP4 sequence is highly conserved between baboon, cynomolgus macaque, and human EP4, the seven putative transmembrane domains are identical between the three species. The conserved amino acids predicted to be embedded in the cell membrane have been shown to have critical roles in ligand binding both for prostaglandin receptors and for other G protein-coupled receptors with small molecular weight ligands. Two potential sites of N-linked glycosylation at Asn7 in the N-terminal extracellular domain, and Asn177 in the second extracellular loop are present in the predicted ba/cynEP4 sequence and are conserved in the reported human EP4 sequence. N-linked glycosylation of residues in the N-terminal domain and second extracellular loop of the mouse EP3 receptor has been shown to affect the affinity and specificity of ligand-binding (Huang and Tai, *Prostaglandins, Leukotrienes, and Essential Fatty Acids,* 59:265-71 (1998)). Although the EP4 receptors have not yet been demonstrated to be glycoproteins, glycosylation of the N-terminal domain of the β2-adrenergic receptor has been shown to affect targeting of the receptor to the cell surface (Savarese et al., *Biochem. J.,* 283:1-19 (1992)).

A seven amino acid sequence (PG/DTWCFI) in the extracellular loop 2 of the ba/cynEP4 is present in all reported PGE₂ receptors (EP1-EP4) of the human, mouse, rat, dog, and rabbit. This suggests a fundamental role for the sequence PG/DTWCFI in ligand recognition. For example, mutational analysis of this region in the rabbit EP3 receptor shows that substitution for the proline, tryptophan, or threonine residues in this sequence results in dramatically altered ligand specificity (Audoly and Breyer, *J. Biol. Chem.,* 272:13475-78 (1997)). Also, although not highly conserved, residues in the N-terminal domain of the EP2 and EP4 prostaglandin receptors have been shown to have a similar functional role in determination of ligand specificity (Stillman et al., *Mol. Pharm.,* 56:545-51 (1999)).

Also conserved between the predicted ba/cynEP4 and human EP4 protein sequences are serine residues at positions 45 (intracellular loop 1), 261 (intracellular loop 3) and 356, 366, 432, 462, and 488 (C-terminal intracellular domain). In addition, a threonine residue at position 435 in the C-terminal intracellular domain is conserved in both ba/cynEP4 and human EP4. These serine and threonine residues are potential sites of phosphorylation by protein kinase C and are also conserved between the ba/cynEP4 and human EP4 receptors. Since phosphorylation of serine and threonine residues in the C-terminal domain of the β2-adrenergic receptor is thought to be involved in the control of receptor desensitization, these six residues may be involved in the desensitization seen in the ba/cynEP4 receptor (Inglese et al., *J. Biol. Chem.,* 268:23735-38 (1993)).

To test the binding and signaling characteristics of this putative ba/cynEP4 receptor, DNA fragments containing the entire open reading frames of each baboon and cynomolgus macaque were separately transiently transfected into CHO-K1 cells. The ligand binding specificities of these receptors are illustrated in FIGURES 4 and 5. ³H-PGE₂ binding was displaced by PGE₂ (IC₅₀ 2.6 nM for baboon and 2.5 nM for cynomolgus macaque) and EP4SL (IC₅₀ 361 nM for baboon and 300 nM for cynomolgus macaque). In contrast ligands selective for the related prostanoid receptors EP1/EP3 (sulprostone) and EP2 (butaprost) did not bind to this EP4 receptor. There was no specific binding to untransfected cells.

Secondary signaling in CHO cells expressing baEP4 or cynEP4 was measured by cAMP accumulation. CHO cells transiently transfected with baEP4 or cynEP4 were treated for 12 minutes with various concentrations of PGE₂ in the presence of 2mM IBMX. Accumulation of intracellular cAMP was measured at the end of the treatment period by use of a commercially available scintillation proximity assay (SPA) kit (Amersham, Arlington Heights, IL). The EC50 for cAMP accumulation following PGE₂ treatment was 22.4 nM for baboon and 8.9 nM for cynomolgus macaque, and the EC50 following treatment with the EP4 selective ligand EP4SL was 872 nM for baboon and 560 nM for cynomolgus macaque (FIGURES 6 and 7).

### Definitions

"Proteinaceous molecule" generally encompasses any molecule made up of a plurality of amino acids. The term is broad enough to include peptides, oligopeptides, and proteins. Typically, the amino acids in the proteinaceous material will be selected from the 20 naturally occurring amino acids. However, amino acid analogs and derivatives could also be included in the proteinaceous molecule. The proteinaceous molecule will usually be made up of about the same number of amino acids contained in naturally occurring ba/cynEP4 (i.e., about 490 amino acids). In addition, however, the proteinaceous molecule may have a greater or lesser number of amino acids, as long as the molecule retains an activity of ba/cynEP4. This activity need not be quantitatively the same as the activity of the natural protein, and may be more or less than that activity, as long as it is measurable by an assay of ba/cynEP4 activity (see below). Preferably, such molecules will possess at least about 50% of the activity of the naturally occurring ba/cynEP4. In some hosts, expression of recombinant ba/cynEP4 will result in a protein having an N-terminal methionine residue. Amino acid sequences containing such a residue on the N-terminus thereof are also within the scope of the present invention.

By "conservative substitution" is meant a substitution, addition, or deletion of an amino acid in a proteinaceous molecule that is expected to have little or no affect on the activity or expression thereof. For example, the replacement of one hydrophobic amino acid for another in a transmembrane region of a proteinaceous molecule will seldom have any significant impact on the activity of thereof. Other conservative substitutions will be well known to those skilled in the art in light of this disclosure.

By "activity of ba/cynEP4" is meant any activity that is measurable by an in vivo or in vitro ba/cynEP4 assay. Qualitatively, the activity will generally be one that is possessed by the naturally occurring ba/cynEP4 protein. Cells expressing, or thought to be expressing, ba/cynEP4 protein may be assayed for both the levels of ba/cynEP4 receptor activity and levels of ba/cynEP4 protein. Assessing ba/cynEP4 receptor activity preferably involves the direct introduction of a labeled ligand to the cells and determining the amount of specific binding of the ligand to the ba/cynEP4-expressing cells. Binding assays for receptor activity are known in the art (Sando et al., *Biochem. Biophys. Res. Comm.,* 200:1329-1333 (1994)). Levels of ba/cynEP4 protein in host cells may be quantitated by a variety of techniques including, but not limited to, immunoaffinity and/or ligand affinity techniques. ba/cynEP4-specific affinity beads or ba/cynEP4-specific antibodies can be used to isolate ³⁵S-methionine labeled or unlabeled ba/cynEP4 protein. Labeled ba/cynEP4 protein may be analyzed by SDS-PAGE. Unlabeled ba/cynEP4 protein may be detected by Western blotting, ELISA, or RIA assays employing ba/cynEP4-specific antibodies.

By "isolated" is meant that a substance is removed from its naturally occurring environment. In preferred forms, "isolated" means that a substance is substantially free from normally occurring impurities or other molecules, especially other proteinaceous molecules, salts, other cellular constituents, and the like. Isolation may typically be carried out by standard methods in the art of nucleotide, peptide, and/or protein purification or synthesis. Following expression of ba/cynEP4 in a host cell, ba/cynEP4 protein may be recovered to provide ba/cynEP4 in active form, capable of binding ba/cynEP4-specific ligands. Several ba/cynEP4 purification procedures are available and suitable for use. Recombinant ba/cynEP4 may be purified from cell lysates and extracts, or from conditioned culture medium, by various combinations of, or individual application of salt fractionation, ion exchange chromatography, size exclusion chromatography, hydroxylapatite adsorption chromatography, and hydrophobic interaction chromatography. In addition, recombinant ba/cynEP4 can be separated from other cellular proteins by use of an immunoaffinity column made with monoclonal or polyclonal antibodies specific for full length nascent ba/cynEP4, or polypeptide fragments of ba/cynEP4.

"Polynucleotide sequences" encompass both DNA- and RNA-containing molecules. The DNA molecules will preferably be intronless sequences (i.e., cDNA), but can contain enhancer sequences, termination sequences, and the like, to facilitate or increase expression in a particular host.

By "recombinant expression vector" is meant a vector (e.g., a plasmid or λ phage) that is capable of transferring polynucleotide sequences contained therein into cells of a host organism for expression of the transferred sequences. The sequences are operably linked to other sequences capable of effecting or modulating their expression. Such expression vectors must be replicable in a host organism. For example, any DNA sequence that is capable of effecting expression of a specified DNA sequence disposed therein is included in this term as it is applied to the specified sequence.

The "cells" that may be transformed by way of the vectors described above are those that are capable of expressing the polynucleotide sequences that have been transferred by the vector. Culturing conditions for such cells may be those standard in the art of recombinant protein production.

"Specific binding partners" are molecules that are capable, on a molecular level, of recognizing and interacting with the proteinaceous or polynucleotide molecules described herein. Included within this term are immunological binding partners such as antibody molecules, antigen-binding fragments of antibodies (e.g., Fab and F(ab')₂ fragments), single chain antigen-binding molecules, and the like, whether produced by hybridoma or rDNA technology. Other proteinaceous or non-proteinaceous binding partners are also included within the broad term.

### Proteinaceous Molecules

The proteinaceous molecules of this invention will now be described in greater detail. SEQ ID NO:3 corresponds to the naturally occurring baboon/cynomolgus macaque peptide having 490 amino acids.

The family of ba/cynEP4 proteins provided herein also includes proteinaceous molecules in which one or more of the amino acids in the above-recited amino acid sequences has been deleted, modified, or changed to another amino acid. Site directed mutagenesis is a preferred technique enabling conversion of one amino acid to another. For example, one or more of the cysteine residues may be changed to another amino acid such as serine. One possible reason for such a change would be to eliminate one or more unwanted disulfide bonds. See, for example, U.S. Pat. No. 4,518,584.

Other contemplated specific changes in the natural amino acid sequences involve modification of the asparagine glycosylation site. Modification of the asparagine (Asn-7) or of one or both of the subsequent two amino acids (alanine and serine in SEQ ID NO:3) can eliminate glycosylation at the modified site. Thus, for example, the asparagine could be changed to a glutamine, thereby eliminating glycosylation at the site. See, for example, Miyajima et al., *EMBO J.,* 5(6):1993 (1986).

To determine the ba/cynEP4 cDNA sequence(s) that yields optimal levels of receptor activity and/or ba/cynEP4 protein, ba/cynEP4 cDNA molecules including but not limited to the following can be constructed: the full-length open reading frame of the ba/cynEP4 cDNA and various constructs containing portions of the cDNA encoding only specific domains of the receptor protein or rearranged domains of the protein. All constructs can be designed to contain none, all, or portions of the 5' and/or 3' untranslated region of ba/cynEP4 cDNA. ba/cynEP4 activity and levels of protein expression can be determined following the introduction, both singly and in combination, of these constructs into appropriate host cells. Following determination of the ba/cynEP4 cDNA cassette yielding optimal expression in transient assays, this ba/cynEP4 cDNA construct is transferred to a variety of expression vectors (including recombinant viruses), including but not limited to those for mammalian cells, plant cells, insect cells, oocytes, E. coli, and yeast cells.

The modifications of the amino acid sequences described above that form a part of the present invention are those that result in proteinaceous molecules having an activity of baboon and cynomolgus macaque EP4. Such activity may be determined using the assays described herein, or equivalent assays now known or yet to be developed. The family of proteinaceous molecules of the present invention will also generally be highly homologous to SEQ ID NO:3, which means that several of the amino acids in each sequence may be deleted, modified, or changed (assuming the resulting proteinaceous material retains at least some activity in common with baboon and cynomolgus macaque EP4).

The proteinaceous molecules of the present invention may further be labeled by attachment to a detectable marker substance (e.g., radiolabeled with ¹²⁵I) to provide reagents useful in vitro or in vivo.

### Polynucleotide Sequences

The present invention is also directed to polynucleotide sequences. Preferred polynucleotide sequences are DNA molecules that encode the proteinaceous molecules of the present invention, described above. Preferred DNA sequences (SEQ ID NO:1 and 2) are shown in FIGURES 1 and 2 herein. The cDNAs of FIGURES 1 and 2 are described in greater detail herein.

It is to be recognized that more than one DNA sequence can encode the same amino acid sequence, due to the degeneracy of the genetic code. All such sequences are encompassed by the polynucleotide sequences described herein.

The presently preferred method of obtaining the cDNAs of FIGURES 1 or 2 is described in the Examples Section below. However, any of a variety of alternative procedures may be used to clone ba/cynEP4 cDNA. These methods include, but are not limited to, direct functional expression of the ba/cynEP4 cDNA following the construction of a ba/cynEP4-containing cDNA library in an appropriate expression vector system. Another method is to screen an ba/cynEP4-containing cDNA library constructed in a bacteriophage or plasmid shuttle vector with a labeled oligonucleotide probe designed from the amino acid sequence of the ba/cynEP4 protein. The preferred method consists of screening a ba/cynEP4-containing cDNA library constructed in a bacteriophage or plasmid shuttle vector with a partial cDNA encoding the ba/cynEP4 protein. This partial cDNA is obtained by the specific PCR amplification of ba/cynEP4 DNA fragments through the design of degenerate oligonucleotide primers from the amino acid sequence known for other G protein-coupled receptors which are related to the prostaglandin ba/cynEP4 receptors.

It will be readily apparent to those skilled in the art in light of this disclosure that other types of libraries, as well as libraries constructed from other cells or cell types, may be useful for isolating ba/cynEP4-encoding DNA. Other types of libraries include, but are not limited to, cDNA libraries derived from other cells or cell lines other than baboon and cynomolgus macaque spleen and heart cells, and genomic DNA libraries.

It will be readily apparent to those skilled in the art in light of this disclosure that suitable cDNA libraries may be prepared from cells or cell lines that have ba/cynEP4 activity. The selection of cells or cell lines for use in preparing a cDNA library to isolate ba/cynEP4 cDNA may be done by first measuring cell associated ba/cynEP4 activity using the labeled ligand binding assay cited herein.

Preparation of cDNA libraries can be performed by standard techniques well known in the art. Well known cDNA library construction techniques can be found for example, in Maniatis et al., Molecular Cloning: A Laboratory Manual (Cold Spring Harbor Laboratory, Cold Spring Harbor, N.Y., 1982).

It will also be readily apparent to those skilled in the art in light of this disclosure that DNA encoding ba/cynEP4 may also be isolated from a suitable genomic DNA library. Construction of genomic DNA libraries can be performed by standard techniques well known in the art. Well known genomic DNA library construction techniques can be found in Maniatis et al., Molecular Cloning: A Laboratory Manual (Cold Spring Harbor Laboratory, Cold Spring Harbor, N.Y., 1982).

In order to clone the ba/cynEP4 gene by one of the preferred methods, the amino acid sequence or DNA sequence of ba/cynEP4 or a homologous protein is necessary. To accomplish this, ba/cynEP4 protein or a homologous protein may be purified and partial amino acid sequence determined by automated sequenators. It is not necessary to determine the entire amino acid sequence, but the linear sequence of two regions of 6 to 8 amino acids can be determined for the PCR amplification of a partial ba/cynEP4 DNA fragment.

Once suitable amino acid sequences have been identified, the DNA sequences capable of encoding them are synthesized. Because the genetic code is degenerate, more than one codon may be used to encode a particular amino acid, and therefore, the amino acid sequence can be encoded by any of a set of similar DNA oligonucleotides. Only one member of the set will be identical to the ba/cynEP4 sequence but others in the set may be capable of hybridizing to ba/cynEP4 DNA even though they contain mismatches. The mismatched DNA oligonucleotides may still sufficiently hybridize to the ba/cynEP4 DNA to permit identification and isolation of ba/cynEP4 encoding DNA.

Using one of the preferred methods, cDNA clones encoding ba/cynEP4 are isolated in a two-stage approach employing polymerase chain reaction (PCR) based technology and cDNA library screening. In the first stage, NH₂-terminal and internal amino acid sequence information from the purified ba/cynEP4 or a homologous protein is used to design degenerate oligonucleotide primers for the amplification of ba/cynEP4-specific DNA fragments. In the second stage, these fragments are cloned to serve as probes for the isolation of full length cDNA from a cDNA library derived from baboon and cynomolgus macaque heart or spleen cells.

Polynucleotide products of the present invention may be labeled with detectable markers (such as radiolabels and non-isotopic labels such as biotin) and employed, for example, in DNA hybridization processes to locate the baboon or cynomolgus macaque gene position and/or the position of any related gene family in a chromosomal map. They may also be used for identifying baboon or cynomolgus macaque gene disorders at the DNA level and used as gene markers for identifying neighboring genes and their disorders.

### Expression Vectors, Hosts, and Recombinant Methods

The cloned ba/cynEP4 cDNA obtained through the methods described herein may be recombinantly expressed by molecular cloning into an expression vector containing a suitable promoter and other appropriate transcription regulatory elements, and transferred into prokaryotic or eukaryotic host cells to produce recombinant ba/cynEP4. Techniques for such manipulations can be found described in Maniatis et al., supra, and are well known in the art in light of this disclosure.

Expression vectors are defined herein as DNA sequences that are required for the transcription of cloned DNA and the translation of their mRNAs in an appropriate host. Such vectors can be used to express eukaryotic DNA in a variety of hosts such as bacteria, bluegreen algae, plant cells, insect cells, and animal cells.

Specifically designed vectors allow the shuttling of DNA between hosts such as bacteria-yeast or bacteria-animal cells. An appropriately constructed expression vector preferably contains: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one that causes mRNAs to be initiated at high frequency. Expression vectors may include, but are not limited to, cloning vectors, modified cloning vectors, specifically designed plasmids, or viruses.

A variety of mammalian expression vectors may be used to express recombinant ba/cynEP4 in mammalian cells. Commercially or otherwise available mammalian expression vectors that may be suitable for recombinant ba/cynEP4 expression, include but are not limited to, pMClneo (Stratagene, La Jolla, CA), pXT1 (Stratagene), pSG5 (Stratagene), pcDNAI, pcDNAlamp (Invitrogen, Carlsbad, CA), EBO-pSV2-neo (ATCC 37593, Manassas, VA) pBPV-1(8-2) (ATCC 37110), pdBPV-MMTneo(342-12) (ATCC 37224) pRS-Vgpt (ATCC 37199), pRSVneo (ATCC 37198), pSV2-dhfr (ATCC 37146), pUCTag (ATCC 37460), and IZD35 (ATCC 37565)

DNA encoding ba/cynEP4 may also be cloned into an expression vector for expression in a host cell. Host cells may be prokaryotic or eukaryotic, including but not limited to bacteria, yeast, mammalian cells including but not limited to cell lines of human, bovine, porcine, monkey, and rodent origin, and insect cells including but not limited to drosophila derived cell lines. Cell lines derived from mammalian species which may be suitable and which are commercially or otherwise available, include but are not limited to, CV-1 (ATCC CCL 70), COS-1 (ATCC CRL 1650), COS-7 (ATCC CRL 1651), CHO-KI (ATCC CCL 61), 3T3 (ATCC CCL 92), NIHi3T3 (ATCC CRL 1658), HeLa (ATCC CCL 2), Cl271 (ATCC CRL 1616), BS-C-1 (ATCC CCL 26), and MRC-5 (ATCC CCL 171).

The expression vector may be introduced into host cells via any one of a number of techniques including but not limited to transformation, transfection, protoplast fusion, and electroporation. The expression vector-containing cells are individually analyzed to determine whether they produce ba/cynEP4 protein. Identification of ba/cynEP4 expressing cells may be done by several means, including but not limited to immunological reactivity with anti-ba/cynEP4 antibodies, and the presence of host cell-associated ba/cynEP4 activity.

Expression of ba/cynEP4 DNA may also be performed using in vitro produced synthetic mRNA. Synthetic mRNA can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

A variety of prokaryotic cells known to those of ordinary skill in this art may be utilized. A few exemplary prokaryotes include *E. coli, Bacillus subtilis,* and various strains of *Pseudomonas.*

In addition to bacteria, eukaryotic microbes, such as yeast, may also be used as a host. Laboratory strains of *Saccharomyces cerevisiae* are most commonly used.

It is also possible to express genes encoding polypeptides in eukaryotic host cell cultures derived from multi-cellular organisms. Useful host cell lines include Vero, HeLa, COS, and Chinese hamster ovary (CHO) cells. Expression vectors for such cells ordinarily include promoters and control sequences compatible with mammalian cells such as, for example, the commonly used early and later promoters from Simian Virus 40 (SV40) or other viral promoters such as those derived from polyoma, adenovirus 2, bovine papilloma virus, avian sarcoma viruses, immunoglobulin promoters, and heat shock promoters. Enhancer regions may also be included as desired.

Other examples of hosts, vectors, enhancers, promoters, etc., may be found in the following exemplary U.S. Pat. Nos. 4,810,643; 4,766,075; and 4,847,201, each of which is incorporated by reference herein.

### Specific Binding Partners

Specific binding partners directed to the proteinaceous molecules and polynucleotides of the present invention may be generated by any standard technique known to those of skill in the art. Preferred specific binding partners are immunological binding partners such as intact antibodies and fragments thereof. The immunological binding partners are preferably monoclonal antibodies directed to a specific antigen, which are prepared by standard techniques of monoclonal antibody production.

These specific binding partners may be utilized, for example, to purify the proteinaceous materials or polynucleotides of the present invention. Specific binding partners in labeled form may be utilized to indicate the presence of the proteinaceous molecules or polynucleotides of the present invention. In one preferred embodiment, a specific binding partner of a polynucleotide of the present invention may be utilized in labeled form to locate the natural gene on a chromosome.

Monospecific antibodies to ba/cynEP4 are purified from mammalian antisera containing antibodies reactive against ba/cynEP4 or are prepared as monoclonal antibodies reactive with ba/cynEP4 using the technique of Kohler and Milstein, *Nature,* 256:495-497 (1975). Monospecific antibody as used herein is defined as a single antibody species or multiple antibody species with homogenous binding characteristics for ba/cynEP4. Homogenous binding as used herein refers to the ability of the antibody species to bind to a specific antigen or epitope, such as those associated with the ba/cynEP4, as described above. ba/cynEP4 specific antibodies are raised by immunizing animals such as mice, rats, guinea pigs, rabbits, goats, horses, and the like, with an appropriate concentration of ba/cynEP4 either with or without an immune adjuvant.

Preimmune serum is collected prior to the first immunization. Each animal receives between about 0.1 µg and about 1000 µg of ba/cynEP4 associated with an acceptable immune adjuvant. Such acceptable adjuvants include, but are not limited to, Freund's complete, Freund's incomplete, alum-precipitate, water in oil emulsion containing *Corynebacterium parvum,* and tRNA. The initial immunization consists of the proteinaceous molecule in, preferably, Freund's complete adjuvant at multiple sites either subcutaneously (SC), intraperitoneally (IP), or both. Each animal is bled at regular intervals, preferably weekly, to determine antibody titer. The animals may or may not receive booster injections following the initial immunization. Those animals receiving booster injections are generally given an equal amount of ba/cynEP4 in Freund's incomplete adjuvant by the same route. Booster injections are given at about three week intervals until maximal titers are obtained. At about 7 days after each booster immunization or about weekly after a single immunization, the animals are bled, the serum collected, and aliquots are stored at about -20° C.

Monoclonal antibodies (mAb) reactive with ba/cynEP4 are prepared by immunizing inbred mice, preferably Balb/c, with ba/cynEP4. The mice are immunized by the IP or SC route with about 1 µg to about 100 µg, preferably about 10 µg, of ba/cynEP4 in about 0.5 ml buffer or saline incorporated in an equal volume of an acceptable adjuvant, as discussed above. Freund's complete adjuvant is preferred. The mice receive an initial immunization on day 0 and are rested for about 3 to about 30 weeks. Immunized mice are given one or more booster immunizations of about 1 to about 100 µg of ba/cynEP4 in a buffer solution such as phosphate buffered saline by the intravenous (IV) route. Lymphocytes, from antibody positive mice, preferably splenic lymphocytes, are obtained by removing spleens from immunized mice by standard procedures known in the art. Hybridoma cells are produced by mixing the splenic lymphocytes with an appropriate fusion partner, preferably myeloma cells, under conditions which will allow the formation of stable hybridomas. Fusion partners may include, but are not limited to: mouse myelomas P3/NS1/Ag 4-1, MPC-11, S-194, and Sp 2/0, with Sp 2/0 being preferred (all available from ATCC, Manassas, VA). The antibody producing cells and myeloma cells are fused in polyethylene glycol, about 1000 mol. wt., at concentrations from about 30% to about 50%. Fused hybridoma cells are selected by growth in hypoxanthine, thymidine, and aminopterin supplemented Dulbecco's Modified Eagles Medium (DMEM) by procedures known in the art. Supernatant fluids are collected from growth positive wells on about days 14, 18, and 21 and are screened for antibody production by an immunoassay such as solid phase immunoradioassay (SPIRA) using ba/cynEP4 as the antigen. The culture fluids are also tested in the Ouchterlony precipitation assay to determine the isotype of the mAb. Hybridoma cells from antibody positive wells are cloned by a technique such as the soft agar technique of MacPherson, (Soft Agar Techniques, in Tissue Culture Methods and Applications, IQ-use and Paterson, Eds., Academic Press, 1973).

Monoclonal antibodies are produced in vivo by injection of pristine primed Balb/c mice, approximately 0.5 ml per mouse, with about 2x10⁶ to about 6x10⁶ hybridoma cells about 4 days after priming. Ascites fluid is collected at approximately 8-12 days after cell transfer and the monoclonal antibodies are purified by techniques known in the art.

In vitro production of anti-ba/cynEP4 mAb is carried out by growing the hydridoma in DMEM containing about 2% fetal calf serum to obtain sufficient quantities of the specific mAb. The mAb are purified by techniques known in the art.

Antibody titers of ascites or hybridoma culture fluids are determined by various serological or immunological assays which include, but are not limited to, precipitation, passive agglutination, enzyme-linked immunosorbent antibody (ELISA) technique, and radioimmunoassay (RIA) techniques. Similar assays are used to detect the presence of ba/cynEP4 in body fluids or tissue and cell extracts.

It will be readily apparent to those skilled in the art in light of this disclosure that the above described methods for producing monospecific antibodies may be utilized to produce antibodies specific for ba/cynEP4 polypeptide fragments or full-length ba/cynEP4 polypeptide. ba/cynEP4 antibody affinity columns are made by adding the antibodies to Affigel-10 (Biorad, Hercules, CA), a gel support that is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1 M ethanolamine HCI (pH 8). The column is washed with water followed by 0.23M glycine HCI (pH 2.6) to remove any non-conjugated antibody or extraneous protein. The column is then equilibrated in phosphate buffered saline (pH 7.3) and the cell culture supernatants or cell extracts containing ba/cynEP4 or ba/cynEP4 fragments are slowly passed through the column. The column is then washed with phosphate buffered saline until the optical density (A₂₈₀) falls to background, then the protein is eluted with 0.23M glycine-HCI (pH 2.6). The purified ba/cynEP4 protein is then dialyzed against phosphate buffered saline.

The novel baboon and cynomolgus macaque prostaglandin receptors of the present invention are suitable for use in an assay procedure for the identification of compounds that modulate the receptor activity. Modulating receptor activity, as described herein, includes the inhibition or activation of the receptor and also includes directly or indirectly affecting the normal regulation of the receptor activity. Compounds that modulate the receptor activity include agonists, antagonists, and compounds which directly or indirectly affect regulation of the receptor activity.

The baboon and cynomolgus macaque prostaglandin receptors of the present invention may be obtained from both native and recombinant sources for use in an assay procedure to identify receptor modulators. In general, an assay procedure to identify baboon and cynomolgus macaque prostaglandin receptor modulators will comprise the baboon or cynomolgus macaque prostaglandin receptor of the present invention, and a test compound or sample which contains a putative prostaglandin receptor modulator. The test compounds or samples may be tested directly on, for example, purified receptor protein whether native or recombinant, subcellular fractions of receptor-producing cells whether native or recombinant, and/or whole cells expressing the receptor whether native or recombinant. The test compound or sample may be added to the receptor in the presence or absence of a known labeled or unlabeled receptor ligand. The modulating activity of the test compound or sample may be determined by, for example, analyzing the ability of the test compound or sample to bind to the receptor, activate the receptor, inhibit receptor activity, inhibit or enhance the binding of other compounds to the receptor, modifying receptor regulation, or modifying an intracellular activity. According to the present invention a method of determining if a test substance is a ligand for the ba/cynEP4 protein, may comprise the steps of:
a) contacting the test substance with an isolated proteinaceous molecule having ba/cynEP4 activity, wherein the proteinaceous molecule has previously been combined with a detectable ligand;
b) measuring the amount of detectable ligand that is competitively inhibited from binding with the proteinaceous molecule by the test substance; and
c) classifying as a ligand the test substance if it competitively inhibits the binding of the detectable ligand with the proteinaceous molecule.

The identification of modulators of ba/cynEP4 receptor activity are useful in treating disease states involving the ba/cynEP4 receptor activity. Potentially, these compounds are also modulators of homologous EP4 receptors from other species, and will be useful in treating disease states in these other species as well. Other compounds may be useful for stimulating or inhibiting activity of the receptor. These compounds could be useful as antiinflammatory and antipyretic agents, as analgesics, and as means of stimulating or inhibiting bone formation. Such compounds could be of use in the treatment of diseases in which activation of the ba/cynEP4 receptor results in either cellular proliferation, induction of cellular neoplastic transformations, or metastatic tumor growth, and hence could be used in the prevention and/or treatment of cancers such as colon cancer. The isolation and purification of an ba/cynEP4-encoding DNA molecule would be useful for establishing the tissue distribution of ba/cynEP4 receptors as well as establishing a process for identifying compounds which modulate ba/cynEP4 receptor activity.

The invention now being generally described, the same will be better understood by reference to certain specific examples which are included herein for illustrative purposes only and are not intended to be limiting of the present invention, except where so stated.

### EXAMPLES

### Example 1 - cDNA library screening

For the baboon, two cDNA libraries, one constructed from baboon spleen and one constructed from baboon heart, were obtained from a commercial source (Stratagene, San Diego, CA). cDNA clones in these libraries were converted to plasmid clones using protocols available from the manufacturer. Bulk plasmid DNA was prepared from 500 ml of bacterial culture from each library using the QIAprep Spin kit according to manufacturer's protocols (Qiagen, Valencia, CA). For the cynomolgus macaque, a reverse transcriptase-polymerase chain reaction (RT-PCR) strategy was used to clone the cynEP4 from cynomolgus macaque spleen and heart RNA. The Advantage One-Step RT-PCR kit (Clontech, Palo Alto, CA) was employed to generate cDNA and used 1 g total RNA from each cynomolgus macaque tissue and both random hexamers and oligo-dT primers in a 1 hr reaction at 50°C. From this point forward, both baboon and cynomolgus macaque libraries were treated similarly.

Degenerate primers for use in polymerase chain reaction (PCR) and corresponding to the beginning (5' PO₄-CGMSRRCCACTYTC**ATG**TCC 3' [SEQ ID NO:4]) and end (5' GTGAAACACTGAACTTATCAGAAAAATGTATA**TAR**TAG 3' [SEQ ID NO:5]) of the DNA sequences encoding the human, rat, mouse, dog, and rabbit EP4 receptor protein-coding regions were synthesized (Life Technologies, Gaithersburg, MD). Two independent PCR reactions were performed on the bulk plasmid DNA from the baboon spleen and heart libraries using the following cycling parameters: 5 min at 94°C, 35 cycles of 94°C for 30 sec, 65°C for 30 sec, and 68°C for 3 min followed by a final extension at 68°C for 7 min. Two independent PCR reactions were performed on the cDNA reverse transcribed from the cynomolgus macaque spleen and heart RNA using the following cycling parameters: 5 min at 94°C, 35 cycles of 94°C 30 sec, 65°C for 30 sec, and 68°C for 3 min followed by a final extension at 68°C for 7 min.

Since the 5' primer was phosphorylated at the 5' end, the product of the PCR reaction for each tissue was ligated into the pCR3.1-Uni® vector (Invitrogen, Carlsbad, CA) in a 10I reaction at 15°C overnight. Use of this vector enabled the directional cloning of the PCR generated EP4 protein coding region with respect to the CMV promoter in the vector. Following ligation, each ligation reaction was diluted to 501 and 5I of each reaction was transformed into competent *E. coli* DH5 cells (Life Technologies) according to manufacturer's instructions and the transformed cells plated on LB plates containing 100g/ml ampicillin. 3 ml cultures were initiated from these ampicillin-resistant colonies and plasmid DNA prepared using the QiaQuik kit (Qiagen, Valencia, CA). Restriction enzyme analysis was performed to identify clones with anticipated insert size (∼1500 bp) and four clones from each independent PCR reaction from each tissue were subjected to full-length DNA sequencing. DNA and protein sequence comparisons were performed using Geneworks™ (Oxford Molecular Group, Inc, Campbell, CA).

Sequencing of purified DNA was performed on an AB 3700 DNA Analyzer (PE Biosystems, Foster City, CA) with cycle sequencing using BigDye Terminator (BDT) Taq FS chemistry according to the manufacturer's protocol with the following modifications: Half-reaction BDT reactions (50% BDT, 50% ABI 5X Buffer) contained 5% DMSO (Fischer, Fair Lawn, NJ); cycle sequencing thermal profile with hot start: 95°C for 1 minute for 1 cycle, 98°C for 45 seconds, 50°C for 10 seconds, 60°C for 4 minutes for one cycle, followed by 98°C for 15 seconds, 50°C for 10 seconds, 60°C for four minutes for a total of 30 cycles (MJ Tetrad Thermal Cycler, MJ Research, Watertown, MA). Sequencing reactions were purified by isopropanol (J. T. Baker, Phillipsburg, NJ) precipitation and resuspended in distilled deionized water (VWR, Westchester, PA) for injection.

### Example 2 - Expression of baboon and cynomolgus macaque EP4 in CHO-K1 cells

The day prior to transfection, CHO-K1 cells were plated in 100 mm dishes (Corning, Corning, NY) at 6x10⁴ cells/cm² in DMEM/F12 (Life Technologies) supplemented with 10% fetal calf serum (FCS; Gemini Bio-Products, Calabasas, CA). The next day, each plate of cells was transfected with 15g of either baboon or cynomolgus macaque EP4-pCR3.1-Uni plasmid DNA using Lipofectamine 2000 reagent (Life Technologies) according to the manufacturer's protocol. Briefly, 451 of Lipofectamine 2000 reagent was diluted into 1.5ml of D-MEM/F12 without serum and incubated at room temperature for 5 minutes. During this time, 15g of either baboon or cynomolgus macaque EP4-pCR3.1-Uni plasmid DNA was diluted into 0.5ml of D-MEM/F12 without serum. The diluted Lipofectamine 2000 reagent and the DNA were then mixed and incubated an additional 20 minutes at room temperature. 8ml D-MEM/F12 without serum was then added to each reaction and carefully added to a plate of CHO cell previously rinsed with serum free D-MEM/F12. These cells were then incubated at 37°C with 5% CO₂ for 6 hr at which time 1 ml FCS was added per plate and the incubation continued at 37°C with 5% CO₂ overnight. The following day, the cells were split to either 24-well or 96-well dishes for use in ligand binding and/or secondary signaling analyses.

### Example 3 - Analysis of binding to baboon EP4 and cynomolgus macaque EP4

CHO cells transiently transfected with the baboon EP4 or cynomolgus macaque EP4 receptor were plated at 60,000 cells/well in 24-well tissue-culture dishes (Corning, Corning, NY) and allowed to grow for 48 hours. Whole cell binding reactions (0.2 ml per well) containing 3 nM ³H-PGE₂ plus unlabeled competitor (10⁻⁵ to 10⁻¹⁰ M) were performed on ice for 1 hr. Unbound radioligand was removed by extensive washing, the cells were solubilized with 1% SDS, and the bound ³H-PGE₂ quantitated by scintillation counting.

### Example 4 - Determination of cyclic AMP (cAMP)

To measure stimulation of cAMP production, CHO cells transiently transfected with the baboon EP4 or cynomolgus macaque EP4 receptor were plated at 20,000 cells/well in poly-D lysine coated 96-well tissue-culture plates (Becton-Dickinson, Franklin Lakes, NJ) and allowed to grow for 72 hours. Cells were then rinsed once with PBS and the appropriate concentrations of test compounds (diluted in assay buffer: 5mM MgCl₂; 30 mM Hepes pH 7.4; 0.3 mM 3-isobutyl-1-methylxanthine, (IBMX; Calbiochem, La Jolla, CA); 1 mg/ml dextrose) were added. The plate was incubated at 37°C for 12 minutes, after which 10 µl lysis buffer was added to each well to terminate the reaction. SPA reagent (Amersham:RPA559) was prepared according to the manufacturer's protocol and 120 ml was added to each well. Assay plates were then counted in a Wallac Trilux counter (Perkin Elmer, Boston, MA).

All publications and patents mentioned in the above specification are herein incorporated by reference. Various modifications and variations of the described invention will be apparent to those skilled in the art without departing from the scope and spirit of the invention. Although the invention has been described in connection with specific preferred embodiments, it should be understood that the invention as claimed is not limited to such specific embodiments.

SEQUENCE LISTING

<110> Pfizer Products Inc.

<120> DNA ENCODING THE BABOON AND CYNOMOLGUS MACAQUE PROSTAGLANDIN E2 RECEPTOR
EP4 SUBTYPE

<130> PC11826AGPR

<150> 60/322,916
<151> 2001-09-17

<160> 5

<170> PatentIn version 3.1

<210> 1
<211> 1473
<212> DNA
<213> Baboon

<400> 1
atgtccactc ccggggtcaa tgcgtccacc tccttgagcc ccgaccggct gaacagccca 60

gtgaccatcc cggcggtgat gttcatcttc ggggtggtgg gcaacctggt ggccatcgtg 120

gtgctgtgca agtcgcgcaa ggagcagaag gagacaacct tctacacgct ggtatgtggg 180

ctggctgtca ccgacctgtt gggcactttg ctggtgagcc cggtgaccat cgccacgtac 240

atgaagggcc aatggcccgg gggccagccg ctgtgcgagt acagcacctt cattctgctc 300

ttcttcagcc tgtccggcct tagcatcatc tgcgccatga gtgtcgagcg ctacctggcc 360

atcaaccacg cctatttcta cagccactac gtggacaagc ggttggcggg cctcacgctc 420

tttgcagtct atgcgtccaa cgtgctcttc tgcgcgctgc ccaacatggg cctcggtagc 480

tcgcggctgc agtacccaga cacctggtgc ttcatcgact ggaccaccaa cgtgacggcg 540

cacgctgcct actcctacat gtacgccggc ttcagctcct tcctcattct cgccaccgtc 600

ctctgtaacg tgcttgtgtg cggcgcgctg ctccgcatgc accgccagtt catgcgccgc 660

acctcgctgg gcaccgagca gcaccatgcg gccgcgacgg ccgtgacctc ggttgtctcc 720

cggggccacc ccgccgcctc cccaggcttg ccgcgcctca gcgactttcg tcgccgccgg 780

agtttccgcc gcatcgcggg cgccgagatc cagatggtca tcttactcat tgccacctcc 840

ctggtggtgc tcatctgctc catcccgctc gtggtgcgag tattcgtcaa ccagttatat 900

cagccaagtt tggagcgaga agtcagtaaa aatccagatt tgcaggccat ccgaattgct 960

tctgtgaacc ccatcctaga cccctggata tatatcctcc tgagaaagac agtgctcagt 1020

aaagcaatag agaagatcaa atgcctcttc tgccgcattg gtggatcccg cagagagcgc 1080

tccggacagc actgctcgga cagtcgaagg acatcttctg ccatgtcggg ccactctcgc 1140

tccttcttct cccgggagct gaaggagatc agcagtacat ctcagaccct cctgccagac 1200

ctctcactgc ctgacctcag tgaaaatggc cttggaggca ggaatttgct tccaggtgtt 1260

cctggcatgg gcctggccca gaaagacacc acctcgctga ggactttgcg aatatcagag 1320

acctcagact cttcacaggg tcaggactca gagagtgtct tactggtgga tgaggttggt 1380

gggagcggca gggctgggcc tgcccctaag ggagctcccc tgcaagtcac atttcccagt 1440

gaaacactga acttatcaga aaaatgtata tag 1473

<210> 2
<211> 1473
<212> DNA
<213> Macaque

<400> 2
atgtccactc ccggggtcaa tgcgtccacc tccttgagcc ccgaccggct gaacagccca 60

gtgaccatcc cggcggtgat gttcatcttc ggggtggtgg gcaacctggt ggccatcgtg 120

gtgctgtgca agtcgcgcaa ggagcagaag gagacaacct tctacacgct ggtatgtggg 180

ctggctgtca ccgacctgtt gggcactttg ctggtgagcc cggtgaccat cgccacgtac 240

atgaagggcc aatggcccgg gggccagccg ctgtgcgagt acagcacctt cattctgctc 300

ttcttcagcc tgtccggcct tagcatcatc tgcgccatga gtgtcgagcg ctacctggcc 360

atcaaccacg cctatttcta cagccactac gtggacaagc ggttggcggg cctcacgctc 420

tttgcagtct atgcgtccaa cgtgctcttc tgcgcgctgc ccaacatggg cctcggtagc 480

tcgcggctgc agtacccaga cacctggtgc ttcatcgact ggaccaccaa cgtgacggcg 540

cacgctgcct actcctacat gtacgccggc ttcagctcct tcctcattct cgccaccgtc 600

ctctgtaacg tgcttgtgtg cggcgcgctg ctccgcatgc accgccagtt catgcgccgc 660

acctcgctgg gcaccgagca gcaccatgcg gccgcgacgg ccgtgacctc ggttgtctcc 720

cggggccacc ccgccgcctc cccaggcttg ccgcgcctca gcgactttcg tcgccgccgg 780

agtttccgcc gcatcgcggg cgccgagatc cagatggtca tcttactcat tgccacctcc 840

ctggtggtgc tcatctgctc catcccgctc gtggtgcgag tattcgtcaa ccagttatat 900

cagccaagtt tggagcgaga agtcagtaaa aatccagatt tgcaggccat ccgaattgct 960

tctgtgaacc ccatcctaga cccctggata tatatcctcc tgagaaagac agtgctcagt 1020

aaagcaatag agaagatcaa atgcctcttc tgccgcattg gtggatcccg cagagagcgc 1080

tccggacagc actgctcgga cagtcgaagg acatcttctg ccatgtcggg ccactctcgc 1140

tccttcttct cccgggagct gaaggagatc agcagtacat ctcagaccct cctgccagac 1200

ctctcactgc ctgacctcag tgaaaatggc cttggaggca ggaatttgct tccaggtgtt 1260

cctggcatgg gcctggccca gaaagacacc acctcgctga ggactttgcg aatatcagag 1320

acctcagact cttcacaggg tcaggactca gagagtgtct tactggtgga tgaggttggt 1380

gggagcggca gggctgggcc tgcccctaag gggagctccc tgcaagtcac atttcccagt 1440

gaaacactga acttatcaga aaaatgtata tag 1473

<210> 3
<211> 490
<212> PRT
<213> Baboon/Macaque

<400> 3

Met Ser Thr Pro Gly Val Asn Ala Ser Thr Ser Leu Ser Pro Asp Arg
1               5                   10                  15

Leu Asn Ser Pro Val Thr Ile Pro Ala Val Met Phe Ile Phe Gly Val
            20                  25                  30

Val Gly Asn Leu Val Ala Ile Val Val Leu Cys Lys Ser Arg Lys Glu
        35                  40                  45

Gln Lys Glu Thr Thr Phe Tyr Thr Leu Val Cys Gly Leu Ala Val Thr
    50                  55                  60

Asp Leu Leu Gly Thr Leu Leu Val Ser Pro Val Thr Ile Ala Thr Tyr
65                  70                  75                  80

Met Lys Gly Gln Trp Pro Gly Gly Gln Pro Leu Cys Glu Tyr Ser Thr
                85                  90                  95

Phe Ile Leu Leu Phe Phe Ser Leu Ser Gly Leu Ser Ile Ile Cys Ala
            100                 105                 110

Met Ser Val Glu Arg Tyr Leu Ala Ile Asn His Ala Tyr Phe Tyr Ser
        115                 120                 125

His Tyr Val Asp Lys Arg Leu Ala Gly Leu Thr Leu Phe Ala Val Tyr
    130                 135                 140

Ala Ser Asn Val Leu Phe Cys Ala Leu Pro Asn Met Gly Leu Gly Ser
145                 150                 155                 160

Ser Arg Leu Gln Tyr Pro Asp Thr Trp Cys Phe Ile Asp Trp Thr Thr
                165                 170                 175

Asn Val Thr Ala His Ala Ala Tyr Ser Tyr Met Tyr Ala Gly Phe Ser
            180                 185                 190

Ser Phe Leu Ile Leu Ala Thr Val Leu Cys Asn Val Leu Val Cys Gly
        195                 200                 205

Ala Leu Leu Arg Met His Arg Gln Phe Met Arg Arg Thr Ser Leu Gly
    210                 215                 220

Thr Glu Gln His His Ala Ala Ala Thr Ala Val Thr Ser Val Val Ser
225                 230                 235                 240

Arg Gly His Pro Ala Ala Ser Pro Gly Leu Pro Arg Leu Ser Asp Phe
                245                 250                 255

Arg Arg Arg Arg Ser Phe Arg Arg Ile Ala Gly Ala Glu Ile Gln Met
            260                 265                 270

Val Ile Leu Leu Ile Ala Thr Ser Leu Val Val Leu Ile Cys Ser Ile
        275                 280                 285

Pro Leu Val Val Arg Val Phe Val Asn Gln Leu Tyr Gln Pro Ser Leu
    290                 295                 300

Glu Arg Glu Val Ser Lys Asn Pro Asp Leu Gln Ala Ile Arg Ile Ala
305                 310                 315                 320

Ser Val Asn Pro Ile Leu Asp Pro Trp Ile Tyr Ile Leu Leu Arg Lys
                325                 330                 335

Thr Val Leu Ser Lys Ala Ile Glu Lys Ile Lys Cys Leu Phe Cys Arg
            340                 345                 350

Ile Gly Gly Ser Arg Arg Glu Arg Ser Gly Gln His Cys Ser Asp Ser
        355                 360                 365

Arg Arg Thr Ser Ser Ala Met Ser Gly His Ser Arg Ser Phe Phe Ser
    370                 375                 380

Arg Glu Leu Lys Glu Ile Ser Ser Thr Ser Gln Thr Leu Leu Pro Asp
385                 390                 395                 400

Leu Ser Leu Pro Asp Leu Ser Glu Asn Gly Leu Gly Gly Arg Asn Leu
                405                 410                 415

Leu Pro Gly Val Pro Gly Met Gly Leu Ala Gln Lys Asp Thr Thr Ser
            420                 425                 430

Leu Arg Thr Leu Arg Ile Ser Glu Thr Ser Asp Ser Ser Gln Gly Gln
        435                 440                 445

Asp Ser Glu Ser Val Leu Leu Val Asp Glu Val Gly Gly Ser Gly Arg
    450                 455                 460

Ala Gly Pro Ala Pro Lys Gly Ser Ser Leu Gln Val Thr Phe Pro Ser
465                 470                 475                 480

Glu Thr Leu Asn Leu Ser Glu Lys Cys Ile
                485                 490

<210>  4
<211>  20
<212>  DNA
<213>  Artificial

<400>  4
cgmsrrccac tytcatgtcc                                                   20

<210>  5
<211>  38
<212>  DNA
<213>  Artificial

<400>  5
gtgaaacact gaacttatca gaaaaatgta tatartag                               38

## Claims

1. An isolated proteinaceous molecule having ba/cynEP4 activity, wherein the proteinaceous molecule comprises an amino acid sequence of SEQ ID NO:3, or an amino acid sequence of SEQ ID NO:3 with one or more conservative substitutions therein.

2. An isolated proteinaceous molecule according to claim 1, wherein the amino acid sequence has zero to three conservative substitutions therein.

3. An isolated proteinaceous molecule according to claim 1 or claim 2, wherein the amino acid sequence comprises an amino acid sequence of SEQ ID NO:3.

4. An isolated DNA molecule encoding a proteinaceous molecule according to any one of claims 1 to 3.

5. An isolated DNA molecule encoding a proteinaceous molecule according to claim 3.

6. An isolated DNA molecule having the sequence shown in SEQ ID NO:1 or SEQ ID NO: 2.

7. A recombinant expression vector comprising a DNA molecule according to any one of claims 4 to 6.

8. A cell containing an expression vector according to claim 7.

9. A cell according to claim 8, wherein the cell is that of a eukaryote.

10. A method of producing a proteinaceous molecule having an activity of ba/cynEP4, which comprises culturing a cell according to claim 8 for a time sufficient to produce the proteinaceous molecule.

11. A pharmaceutical composition comprising the proteinaceous molecule according to any one of claims 1 to 3 and a pharmaceutically acceptable carrier therefor.

12. A specific binding partner that selectively binds to the proteinaceous molecule according to any one of claims 1 to 3.

13. A method of determining if a test substance is a ligand for the ba/cynEP4 protein, the method comprising the steps of:
a) contacting the test substance with a proteinaceous molecule according to any one of claims 1 to 3;
b) measuring specific binding between the test substance and the proteinaceous molecule; and
c) classifying as a ligand the test substance if it binds to the proteinaceous molecule.

14. A method of determining if a test substance is a ligand for the ba/cynEP4 protein, the method comprising the steps of:
a) contacting the test substance with a proteinaceous molecule according to any one of claims 1 to 3, wherein the proteinaceous molecule has previously been combined with a detectable ligand;
b) measuring the amount of detectable ligand that is inhibited from binding with the proteinaceous molecule by the test substance; and
c) classifying as a ligand the test substance if it inhibits the binding of the detectable ligand with the proteinaceous molecule.

15. A method according to claim 14 wherein the detectable ligand is radioactively labelled PGE2.
